# EUROPEAN PATENT SPECIFICATION

(11) **EP 2 603 140 B1**
(45) Date of publication and mention of the grant of the patent: **01.04.2015**
(21) Application number: 11725745.1
(22) Date of filing: 16.06.2011
(51) Int. Cl.: A61B 5/103

(54) **DEVICE FOR EVALUATING CONDITION OF SKIN OR HAIR**
VORRICHTUNG ZUR BEURTEILUNG DES ZUSTANDES DER HAUT ODER DES HAARES
DISPOSITIF D'ÉVALUATION DE L'ÉTAT DE LA PEAU OU DES CHEVEUX

(30) Priority: 13.08.2010 US 855728
(43) Date of publication of application: 19.06.2013
(73) Proprietor: Unilever PLC, London, Greater London EC4P 0DY (GB); Unilever N.V., 3013 AL Rotterdam (NL)
(72) Inventor: KRISHNAN, Srinivasan, Trumbull, Connecticut 06611 (US); NICHOL, Jamie, Gordon, Arlington, Massachusetts 02476 (US); CICHOWLAS, Bruce, Framingham, Massachusetts 01701 (US)
(74) Representative: Corsten, Michael Allan
(86) International application number: PCT/EP2011/059995
(87) International publication number: WO 2012/019809

(56) References cited:
- WO-A2-2010/049907
- JP-A- 2003 210 416
- KR-A- 20090 131 370
- US-A1- 2007 185 392
- US-A1- 2009 112 071
- US-A1- 2009 318 908

## Description

### BACKGROUND OF THE INVENTION

### Field of the Invention

The invention concerns a hand holdable device for simultaneously measuring multiple parameters defining a person's skin or hair condition.

### The Related Art

Most people do not have perfect skin or hair. Imperfections can cosmetically be treated with an appropriate product. Selection of the appropriate product is often not scientific. Some will provide excellent results, others modest improvement, and still others will simply not be effective. A correct match needs to start with an evaluation of a person's skin or hair condition.

Evaluation systems have been reported in the literature. For instance, US 5 622 692 (Rigg et al.) discloses a hand-held device for measuring skin color at a point of retail sale and recommending a suitable facial foundation. US 5 945112 (Flynn et al.) discloses a method for providing a customized skin foundation product to cover human skin imperfections. The steps include spectrophotometrically measuring a customer's normal skin to obtain normal skin coloration values of lightness, redness and yellowness. These coloration values are then converted through calculation to a modified value determined by a set program. The skin analyzing module is a hand-held spectrophotometer/colorimeter operating with at least one visible light source such as an LED operating in the 400-900 nm wavelength range.

JP 2003 210416 (Wave Cyber KK) describes a skin measuring instrument held within a transparent body. A light source shines illuminating light through the transparent body and an optical detector absorbs reflected light from the contact surface. Water content is measured by way of static capacitance at a skin contact point. Also present within the body is a camera for photographing the skin surface. US 2009/0318908 A1 discloses a skin monitoring device for application near the skin, comprising a processing circuit connecting means and at least one photosensor configured to detect at least one approximate wavelength of light reflected and/or emitted by the skin, wherein a signal receiving circuit is provided for receiving signals from the at least one photosensor.

Although devices to measure properties of skin or hair are known, none deliver a comprehensive evaluation. Better devices are required which simultaneously measure/deliver information on moisture content, blemishes and topography.

### SUMMARY OF THE INVENTION

A device for evaluating skin or hair condition according to claim 1 is provided.

### BRIEF DESCRIPTION OF THE DRAWING

Further advantages and features of the present invention will become more apparent from consideration of the drawing wherein:
Figure 1 is a perspective view of the first embodiment;
Figure 2 is a top plan view of the first embodiment; and
Figure 3 is a diagrammatic representation of the LED arrangement.

### DETAILED DESCRIPTION OF THE INVENTION

Now we have provided a device which can more fully evaluate skin or hair condition. This device combines a hydration meter measuring moisture with a light based system measuring color and topography. The device is highly portable. Moreover, the collected data can via a cord terminated by a Universal Serial Bus (USB) connector be readily downloaded to a computer. A skin or hair value is then calculated based upon the downloaded data.

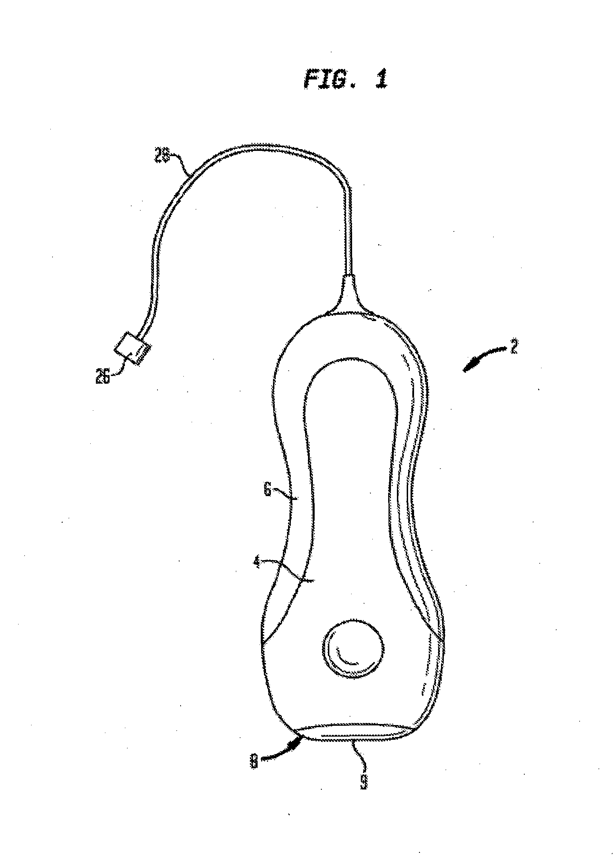

Figure 1 illustrates one embodiment of the hand-held device. By the term "hand-held" is meant a device measuring in length less than 35 cm, preferably between 10 and 25 cm (not including cord) and a width between 2 and 8 inches, preferably between 3 and 6 cm. The device features a housing 2 with a shell 4 and a gripping portion 6. Normally the shell is formed of a relatively hard plastic such as ABS polyacrylonitrile- butadiene-styrene) which is a high impact resistant plastic. Advantageously the gripping area is formed of a less rigid material such as a rubber.

Measurements are taken from an evaluation area 8 of the device. Normally area 8 is on an end of the device distant from any electrical or fiber optic cords exiting the housing. Area 8 has at least a central section formed of a transparent wall. Clear plastic is useful for this purpose. Light transmission through the clear plastic is a necessary requirement.

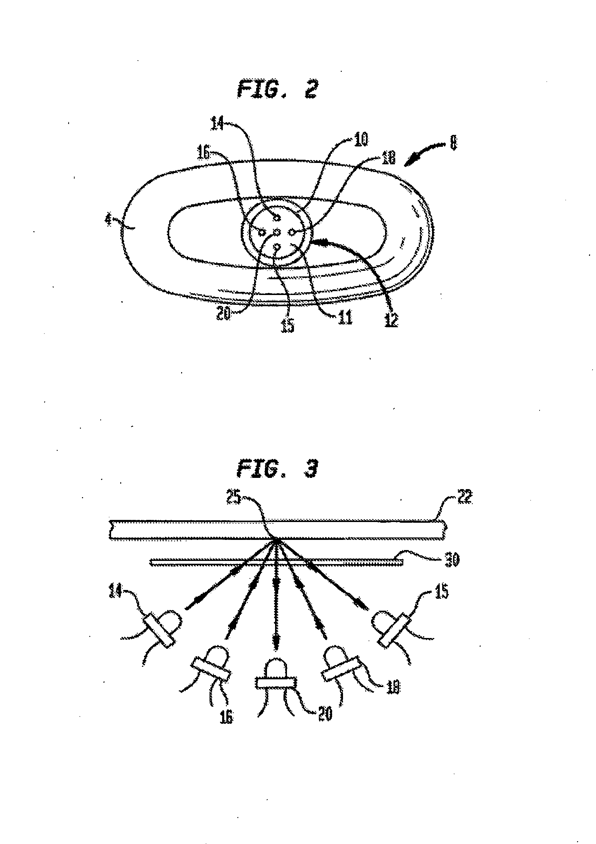

Figure 2 best illustrates the evaluation area 8. When this evaluation area is contacted against skin, the first contact surface is an external surface 9 of a moisture sensing cell 10 of a hydration meter 12. The sensing cell 10 picks up electrical signals from the stratum corneum of skin. Metallic electrical conducting wires, preferably of copper on a circuit board, are embedded within a hardened resin of the sensing cell. These wires are sensitive to differences in dielectric constant of their aqueous environment. Differences in relative electrical capacitance resulting from differences in the dielectric constant reveal the measure of moisture at the skin or hair surface. Hydration meters are commercially available from Courage-Khazaka Electronics, Koln, Germany. The sensing cell 10 is circular (donut shaped) having a central transparent window 11 through which light can be transmitted.

A plurality of light emitting diodes (LEDs) are positioned as a cluster of five within the housing interior to the hydration meter sensing cell 10. These provide input on amount and distribution of melanin. Fig. 2 reveals a red wavelength LED 14 paired with an infrared LED 15; a blue wavelength LED 16; a green wavelength LED 18; and a central absorbing infrared wavelength LED 20. Respective wavelength ranges are for the red (650-560, preferably about 570 nm), green (530-555, preferably about 540 nm), blue (415-440, preferably about 430 nm) and infrared (850-900, preferably about 880 nm). Advantageously the blue, green and red LEDs have their tips angled to transmit light at an angle between 20° and 75°, preferably 35°-55°, and optimally about 45° relative to the central window and to the skin. In other words, these LEDs transmit light at preferentially about a 45° angle to the skin. In a preferred embodiment, the central infrared LED 20 is focused at approximately a 90° orientation (perpendicular) to the central window and to the skin surface.

An important aspect of the present invention is that one or more of the LEDs perform a dual function of both emitting and absorbing reflected light. These LEDs essentially are two-way conduits of light. Their light impinges a skin area 22 and also receives reflection from that area. Fig. 3 best illustrates the LED arrangement.

In the preferred embodiment, reflectance spectroscopy is utilized to evaluate color and texture of the analyzed skin. This means light is bounced off the skin and strength of the reflection is measured. LEDs are utilized because they are photo diodes that can evaluate shorter wavelengths. In the preferred embodiment, one or more of the LEDs emit and detect, but not simultaneously. Some of the LEDs in the device only emit light, others only detect and in the preferred embodiment the central infrared LED performs both functions.

Reflectance spectroscopy measurements can be confused by gloss on the skin surface. The special arrangement of the LEDs in the sensor allows measurement of surface gloss as well as the spectro signature of the skin.

The four LEDs that surround the central infrared LED 20 are arranged so that the axis of each intersects at point 25 where the axis of the central infrared LED impinges at a surface of the skin.

Measurements are taken as follows. The red LED 14 is placed opposite the infrared LED 15. Two measurements of light intensity are taken using the central infrared LED 20 and the infrared LED 15. These two measurements provide input on the spectro/diffuse surface characteristics, and red light amounts the surface absorbs.

Thereafter, the red LED 14 is turned off. Now the green LED 18 is illuminated. A single measurement is taken using the central infrared LED 20. The same sequence is repeated for the blue LED 16 and the infrared LED 15. Almost simultaneously the hydration meter 12 via the moisture sensing cell 10 measures capacitance around or concentric with the illuminated skin surface.

Data generated from the hydration meter and the LEDs initially is in analog form. This form arises because the meter and LEDs are transducers which inherently provide an analog response to the physical event being measured. The analog data is next converted to digital values (numbers stored in a micro-controller). Thereafter, the digital values are transformed back into analog mode, this time as an audio wave signal. The audio signal is thereafter transformed back into a digital signal at a downstream USB microchip. By this manner we can double the base frequency or reduce the base frequency by half. In summary, the data is converted from analog to digital to analog to digital. This ADAD conversion is a key factor allowing use of off-the-shelf components to transfer skin data in a way the computer already recognizes.

The generated data is converted through a pre-set series of calculations to identify a Skin Index unique to the measured skin area. The Skin Index permits a user to monitor their skin over a period of time. A product recommended by the program to adjust the consumer's skin into an improved condition may be applied over the monitored period. This allows a consumer to evaluate effectiveness of the product or any other products that might be applied to improve the skin condition.

A USB port 26 is attached to an end of an electrical wire or optical fiber cord 28 as shown in figure 1. The USB port can plug into a computer to access a program companion to the device. It is the most advantageous connector to the computer. However, the system may also work by Bluetooth connectivity or serial port connection routes.

Most preferred is that power to the system be delivered externally from an electric grid. Alternatively, power can be supplied by a rechargeable battery or disposable batteries within the device. In circumstances wherein power is supplied by a rechargeable or a disposable battery, the only cord projecting from the housing is the data cord 28.

In one aspect of the invention, the device can be considered as a "personal trainer" for a user's skin.

A polarizer plate 30 may be placed adjacent the central window through which all the LEDs transmit/absorb light.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material ought to be understood as modified by the word "about".

It should be noted that in specifying any range of concentration or amount, any particular upper concentration can be associated with any particular lower concentration or amount.

The foregoing description illustrates selected embodiments of the present invention.

## Claims

1. A device for evaluating skin or hair condition comprising:
(i) a housing (2);
(ii) a hydration meter (12) mechanism for measuring moisture; and
(iii) a plurality of light emitting diodes (14,15,16,18, 20) arranged within the housing wherein at least one of the light emitting diodes (20) at different times emits light and detects light reflected from the skin or hair area being evaluated;
**characterized in that**,
the meter is supported within the housing and having an external surface (9) contactable against skin or hair to measure moisture content thereof, the meter comprising at least two adjacent metallic wires with their respective capacitance sensitive to differences in dielectric constant;
wherein the light emitting diodes comprise one red (14), one blue (16), one green (18) and two infrared wavelength light emitters (15, 20);
wherein the external surface portion of the hydration meter surrounds a central window (11), the light emitting diodes being arranged to transmit light through the central window;
wherein the light emitting diodes are angularly oriented to the central window in an angular range from 20° to 90° relative to the central window;
wherein one of the plurality of light emitting diodes is an infrared wavelength receiver oriented perpendicular to the central window (11) and the skin or hair being measured; and
wherein four light emitting diodes (14,15,16,18) surround a central infrared light emitting diode (20) arranged so that the axis of each intersects at a point (25) where the axis of the central infrared light emitting diode impinges at a surface of the skin.

2. A device according to claim 1 wherein the angular range is from 35° to 55°.

3. A device according to claim 1 or claim 2 comprising a single cord (28) bearing an optical fiber or electrical wire for transmission of data and that exits the housing, and at an end of the cord having a USB port (26).

4. A device according to any one of the preceding claims wherein the hydration meter mechanism and light emitting diodes generate data in analog form, and wherein the data in analog form is converted to digital values and then retransformed into an analog form as an audio signal for transmission.

## Patentansprüche

1. Vorrichtung zum Beurteilen eines Haut- oder Haarzustands, die umfasst:
(i) ein Gehäuse (2);
(ii) einen Hydrationsmessgerätmechanismus (12) zum Messen von Feuchtigkeit; und
(iii) mehrere Leuchtdioden (14, 15, 16, 18, 20), die innerhalb des Gehäuses angeordnet sind, wobei mindestens eine der Leuchtdioden (20) zu verschiedenen Zeiten Licht aussendet und von dem beurteilten Haut- oder Haarbereich reflektiertes Licht detektiert;
**dadurch gekennzeichnet, dass**
das Messgerät innerhalb des Gehäuse getragen wird und eine äußere Fläche (9) besitzt, die mit der Haut oder dem Haar in Kontakt gebracht werden kann, um deren oder dessen Feuchtigkeitsgehalt zu messen, wobei das Messgerät mindestens zwei benachbarte Metalldrähte umfasst, wobei ihre jeweiligen Kapazitäten auf Unterschiede in der Dielektrizitätskonstanten empfindlich sind;
wobei die Leuchtdioden einen roten (14), einen blauen (16), einen grünen (18) und zwei Infrarotwellenlängen-Lichtaussender (15, 20) umfassen;
wobei der Abschnitt der äußeren Fläche des Hydrationsmessgerätes ein zentrales Fenster (11) umgibt, wobei die Leuchtdioden ausgelegt sind, Licht durch das zentrale Fenster zu senden;
wobei die Leuchtdioden winkelförmig zu dem zentralen Fenster in einem Winkelbereich von 20° bis 90° bezüglich des zentralen Fensters angeordnet sind;
wobei eine der mehreren Leuchtdioden ein Infrarotwellenlängenempfänger ist, der senkrecht zu dem zentralen Fenster (11) und der gemessenen Haut oder dem gemessenen Haar angeordnet ist; und
wobei vier Leuchtdioden (14, 15, 16, 18) eine zentrale Infrarotleuchtdiode (20) umgeben, die so angeordnet ist, dass die Achse einer jeden einen Punkt (25) schneidet, wo die Achse der zentralen Infrarotleuchtdiode auf die Oberfläche der Haut auftrifft.

2. Vorrichtung nach Anspruch 1, wobei der Winkelbereich 35° bis 55° beträgt.

3. Vorrichtung nach Anspruch 1 oder Anspruch 2, die ein einziges Kabel (28) umfasst, das eine optische Faser oder einen elektrischen Draht für die Übertragung von Daten trägt, und das das Gehäuse verlässt und die an einem Ende de Kabels einen USB-Anschluss (26) besitzt.

4. Vorrichtung nach einem der vorhergehenden Ansprüche, wobei der Hydrationsmessgerätmechanismus und die Leuchtdioden Daten in analoger Form erzeugen und wobei die Daten in analoger Form in digitale Werte umgesetzt werden und dann wieder in eine analoge Form umgewandelt werden, um eine Audiosignal für die Übertragung zu erzeugen.

## Revendications

1. Dispositif d'évaluation de l'état de la peau ou des cheveux comportant .
(i) un boîtier (2) ;
(ii) un mécanisme de mesure du niveau d'hydratation (12) pour mesurer l'humidité ; et
(iii) une pluralité de diodes électroluminescentes (14, 15, 16, 18, 20) disposées dans le boîtier dans lequel au moins une des diodes électroluminescentes (20) émet à des temps différents de la lumière et détecte la lumière réfléchie depuis la peau ou la zone capillaire évaluée ;
**caractérisé en ce que**
le dispositif de mesure est supporté dans le boîtier et possède une surface externe (9) pouvant être en contact avec la peau ou les cheveux pour mesurer la teneur en humidité de ceux-ci, la mesure comprenant au moins deux fils métalliques adjacents ayant leur capacité respective sensible aux différences de constante diélectrique ;
dans lequel les diodes électroluminescentes comprennent une rouge (14), une bleue (16), une verte (18) et deux émetteurs de lumière de longueur d'onde infrarouge (15, 20) ;
dans lequel la partie de surface extérieure du dispositif de mesure d'hydratation entoure une fenêtre centrale (11), les diodes électroluminescentes étant agencées pour transmettre la lumière par l'intermédiaire de la fenêtre centrale ;
dans lequel les diodes électroluminescentes sont orientées angulairement par rapport à la fenêtre centrale d'une plage angulaire allant de 20° à 90° par rapport à la fenêtre centrale ;
dans lequel l'une de la pluralité de diodes électroluminescentes est un récepteur de longueur d'onde infrarouge orienté perpendiculairement à la fenêtre centrale (11) et la peau ou les cheveux mesurés ; et
dans lequel quatre diodes électroluminescentes (14, 15, 16, 18) entourent une diode électroluminescente infrarouge centrale (20) disposée de sorte que l'axe de chacune se croise en un point (25) où l'axe de la diode électroluminescente infrarouge centrale a une incidence sur la surface de la peau.

2. Dispositif selon la revendication 1, dans lequel la plage angulaire va de 35° à 55°.

3. Dispositif selon la revendication 1 ou la revendication 2, comportant un unique cordon (28) supportant une fibre optique ou un fil électrique pour la transmission de données et qui sort du boîtier, et à l'une extrémité du cordon, possède un port USB (26).

4. Dispositif selon l'une quelconque des revendications précédentes, dans lequel le mécanisme de mesure d'hydratation et les diodes électroluminescentes génèrent des données sous forme analogique, et dans lequel les données sous forme analogique sont converties en valeurs numériques et ensuite retransformées sous forme analogique en tant que signal audio pour une transmission.
